(19) Europäisches Patentamt

European Patent Office

Office européen des brevets

(11) **EP 1 731 173 A1**

(12) **EUROPEAN PATENT APPLICATION**
published in accordance with Art. 158(3) EPC

(43) Date of publication:
**13.12.2006 Bulletin 2006/50**

(51) Int Cl.:
*A61K 47/26* (2006.01)   *A61K 9/10* (2006.01)
*A61K 9/127* (2006.01)   *A61K 31/7088* (2006.01)
*A61K 31/713* (2006.01)   *A61K 47/30* (2006.01)
*A61K 47/36* (2006.01)   *A61K 47/42* (2006.01)
*A61K 47/44* (2006.01)   *A61P 35/00* (2006.01)

(21) Application number: 05720512.2

(22) Date of filing: 10.03.2005

(86) International application number:
**PCT/JP2005/004241**

(87) International publication number:
**WO 2005/092389 (06.10.2005 Gazette 2005/40)**

(84) Designated Contracting States:
**AT BE BG CH CY CZ DE DK EE ES FI FR GB GR
HU IE IS IT LI LT LU MC NL PL PT RO SE SI SK TR**

(30) Priority: **10.03.2004 JP 2004067688**

(71) Applicant: **KYOWA HAKKO KOGYO CO., LTD.
Chiyoda-ku,
Tokyo 100-8185 (JP)**

(72) Inventors:
• **YAMAUCHI, Masahiro**

   **(JP)**

• **KATO, Yasuki**

   **(JP)**

(74) Representative: **Teipel, Stephan et al
Lederer & Keller
Patentanwälte
Prinzregentenstrasse 16
80538 München (DE)**

(54) **COMPLEX PARTICLES AND COATED COMPLEX PARTICLES**

(57)   The present invention provides, for example, a method of inhibiting aggregation of complex particles in which a drug is adhered to lead particles, **characterized by** containing a lipid derivative or a fatty acid derivative of one or more substance(s) selected from sugars, peptides, nucleic acids and water-soluble polymers or a surfactant in the lead particles. Further, it provides, for example, a method of producing the complex particles in which a nucleic acid as a drug or a drug is adhered to lead particles, comprising the step of dispersing or dissolving the nucleic acid as a drug or the drug and an adhesion-competitive agent so as to be contained in a liquid in which the lead particles containing a lipid derivative or a fatty acid derivative of one or more substance(s) selected from sugars, peptides, nucleic acids and water-soluble polymers or a surfactant are dispersed, thereby allowing the nucleic acid as a drug or the drug and the adhesion-competitive agent adhered to the lead particles.

EP 1 731 173 A1

## Description

Technical Field

The present invention relates to complex particles and coated complex particles and a method of producing the same.

Background Art

Heretofore, a lot of techniques related to methods of producing coated particles have been disclosed, for pharmaceutical products, foods, agrochemicals, drugs for animals and the like. The coating of particles (particles to be coated) with a coating layer is carried out for imparting a function to particles such as to inhibit an effect given by an external factor, or to selectively receive an effect given by an external factor as a trigger causing change in the particles by the effect.

However, for example, when small particles are coated with a coating layer, the particles are aggregated due to van der Waals forces or electrostatic forces between particles, forces caused by crosslinking of coating layer components or liquid droplets or the like, and coated particles with an undesirable size are obtained in some cases. As the coated particles with an undesirable size, for example, coated particles with a size, which cause clogging of trachea or blood vessels, or are easy to be excreted due to an action of removing foreign matter of the living body and the like in fine particles for transpulmonary administration or fine particles for intravenous injection may be exemplified.

On the other hand, as means for delivering a nucleic acid into a cell, a method using cationic liposome or cationic polymers are known. However, by the method, after a cationic liposome or cationic polymer containing a nucleic acid is intravenously administered, the nucleic acid is promptly removed from the blood, and when a target tissue is other than liver and lung, for example, when it is a tumor site or the like, the nucleic acid cannot be delivered to the target tissue, therefore, it has not been able to achieve the expression of a sufficient action yet. Accordingly, a nucleic acid-encapsulating liposome (liposome encapsulating a nucleic acid therein) by which the problem that a nucleic acid is promptly removed from the blood was solved has been reported (see JP-T-2002-508765, JP-T-2002-501511, "Biochimica et Biophysica Acta", vol. 1510, pp. 152-166 (2001), and Patent document 1). In JP-T-2002-508765, as a method of producing particles containing a nucleic acid or the like, for example, a method of producing an ODN-encapsulating liposome by dissolving a cationic lipid in chloroform in advance, adding an aqueous solution of oligodeoxynucleotide (ODN) and methanol thereto and mixing and centrifuging the mixture thereby transferring a complex of the cationic lipid and ODN to a chloroform layer, and then removing the chloroform layer, adding a polyethylene glycolated phospholipid, a neutral lipid and water to the chloroform layer to form a water-in-oil (w/o) emulsion and treating the emulsion by the reverse phase evaporation method has been reported. In JP-T-2002-501511 and Biochimica et Biophysica Act, a method of producing an ODN-encapsulating liposome by dissolving ODN in an aqueous solution of citric acid at pH 3.8, adding lipid (in ethanol) to the solution, reducing the ethanol concentration to 20 % by volume to prepare an ODN-encapsulating liposome, performing filtration for sizing, removing excess ethanol by dialysis, and then further performing dialysis of the sample at pH 7.5 to remove ODN adhered to the surface of the liposome has been reported. In each method, liposome encapsulating an active ingredient such as a nucleic acid is produced.

On the other hand, in the Patent document 1, it has been reported that liposome encapsulating an active ingredient such as a nucleic acid is produced by a method of coating fine particles with lipid membrane in a liquid. In the method, fine particles are coated with lipid membrane by reducing the ratio of a polar organic solvent in an aqueous solution containing the polar organic solvent in which the fine particles are dispersed and lipid is dissolved. The coating is carried out in the liquid, and for example, coated fine particles with a size suitable for such as fine particles for intravenous injection are produced very efficiently. In addition, a drug complex is exemplified as an example of the fine particles in the Patent document 1. The drug complex is complex particles of lead particles (the same definition as the lead particles described below) and a drug. It has been reported that the particles diameter of coated complex particles obtained by coating the complex particles varies depending on the complex particles to be coated, and coated complex particles obtained by coating complex particles produced by allowing ODN adhered to a cationic liposome of lead particles has a small particles diameter and can be used as an injection, and the coated complex particles shows a high retention in the blood and is accumulated much in a tumor tissue when it is intravenously administered.

On the other hand, siRNA has drawn attention recently as a more effective drug than an antisense drug [see "Biochemical and Biophysical Research Communication", vol. 296, pp. 1000-1004 (2002)]. The blood kinetics of siRNA has not been reported sufficiently so far, however, it is presumed that siRNA promptly disappears from the blood in the same manner as an antisense drug and does not transport to a target tissue. In order to increase the transportation thereof to a target tissue, development of some kind of carrier has been demanded (see "Biochimica et Biophysica Acta", vol. 1281, pp. 139-149 (1996), and "Journal of Controlled Release (J. Controlled Release)", vol. 41, pp. 121-130 (1996)). Patent Document 1: International Application WO 02/28367

Disclosure of the Invention

Problems to be Solved by the Invention

An object of the present invention is to provide a method of inhibiting aggregation of complex particles in which a drug is adhered to lead particles, a method of producing the complex particles and the like. Further, another object of the present invention is to provide a method of producing coated complex particles in which aggregation-inhibited complex particles are coated with a coating layer, coated complex particles that can be produced by the production method and the like.

Means for Solving the Problems

The present invention relates to the following (1) to (47).

(1) A method of inhibiting aggregation of complex particles in which a drug is adhered to lead particles, characterized by containing a lipid derivative or a fatty acid derivative of one or more substance(s) selected from sugars, peptides, nucleic acids and water-soluble polymers or a surfactant in the lead particles.

(2) The method of inhibiting aggregation of complex particles according to the above (1), wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

(3) The method of inhibiting aggregation of complex particles according to the above (1), wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

(4) The method of inhibiting aggregation of complex particles according to any one of the above (1) to (3), wherein the complex particles in which a drug is adhered to lead particles are complex particles obtained by dispersing or dissolving the drug so as to be contained in a liquid in which the lead particles are dispersed and allowing the drug adhered to the lead particles.

(5) The method of inhibiting aggregation of complex particles according to any one of the above (1) to (4), wherein the complex particles in which a drug is adhered to lead particles are complex particles in which a drug electrostatically is adhered to lead particles.

(6) The method of inhibiting aggregation of complex particles according to any one of the above (1) to (5), wherein the lead particles are lead particles having electrostatic charge opposite to that of the drug.

(7) The method of inhibiting aggregation of complex particles according to any one of the above (1) to (6), wherein the lead particles are fine particles containing as a constituent component liposome containing a lipid with electrostatic charge opposite to that of the drug.

(8) The method of inhibiting aggregation of complex particles according to any one of the above (1) to (7), wherein the drug is a nucleic acid.

(9) The method of inhibiting aggregation of complex particles according to the above (8), wherein the nucleic acid as the drug is one or more substance(s) selected from genes, DNA, RNA, oligonucleotides, plasmids and siRNA.

(10) The method of inhibiting aggregation of complex particles according to any one of the above (1) to (9), wherein the complex particles in which a drug is adhered to lead particles are complex particles in which a drug and an adhesion-competitive agent are adhered to lead particles.

(11) The method of inhibiting aggregation of complex particles according to the above (10), wherein the complex particles in which a drug and an adhesion-competitive agent are adhered to lead particles are complex particles obtained by dispersing or dissolving the drug and the adhesion-competitive agent so as to be contained in a liquid in which the lead particles are dispersed and allowing the drug and the adhesion-competitive agent adhered to the lead particles.

(12) The method of inhibiting aggregation of complex particles according to the above (10) or (11), wherein the complex particles in which a drug and an adhesion-competitive agent are adhered to lead particles are complex particles in which a drug and an adhesion-competitive agent are electrostatically adhered to lead particles.

(13) The method of inhibiting aggregation of complex particles according to any one of the above (10) to (12), wherein the adhesion-competitive agent is one or more substance(s) selected from lipids, surfactants, nucleic acids, proteins, peptides and polymers.

(14) The method of inhibiting aggregation of complex particles according to any one of the above (10) to (12), wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.

(15) An inhibitor for aggregation of complex particles in which a drug is adhered to lead particles, containing a lipid derivative or a fatty acid derivative of one or more substance(s) selected from sugars, peptides, nucleic acids and water-soluble polymers or a surfactant.

(16) The inhibitor for aggregation of complex particles according to the above (15), wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

(17) The inhibitor for aggregation of complex particles according to the above (15), wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

(18) A method of producing complex particles in which a nucleic acid as a drug adhered to lead particles, comprising the step of dispersing or dissolving the nucleic acid as a drug so as to be contained in a liquid in which the lead particles containing a lipid derivative or a fatty acid derivative of one or more substance(s) selected from sugars, peptides, nucleic acids and water-soluble polymers or a surfactant are dispersed, thereby allowing the nucleic acid as a drug adhered to the lead particles.

(19) A method of producing complex particles in which a drug is adhered to lead particles, comprising the step of dispersing or dissolving the drug and an adhesion-competitive agent so as to be contained in a liquid in which the lead particles containing a lipid derivative or a fatty acid derivative of one or more substance(s) selected from sugars, peptides, nucleic acids and water-soluble polymers or a surfactant are dispersed, thereby allowing the drug and the adhesion-competitive agent adhered to the lead particles.

(20) The method of producing complex particles according to the above (19), wherein the adhesion-competitive agent is one or more substance(s) selected from lipids, surfactants, nucleic acids, proteins, peptides and polymers.

(21) The method of producing complex particles according to the above (19), wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.

(22) The method of producing complex particles according to any one of the above (19) to (21), wherein the drug is a nucleic acid.

(23) The method of producing complex particles according to the above (18) or (22), wherein the nucleic acid as the drug is one or more substance(s) selected from genes, DNA, RNA, oligonucleotides, plasmids and siRNA.

(24) The method of producing complex particles according to any one of the above (18) to (23), wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

(25) The method of producing complex particles according to any one of the above (18) to (23), wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

(26) The method of producing complex particles according to any one of the above (18) to (25), wherein the lead particles are lead particles having electrostatic charge opposite to that of the drug.

(27) The method of producing complex particles according to any one of the above (18) to (25), wherein the lead particles are fine particles containing as a constituent component liposome containing a lipid with electrostatic charge opposite to that of the drug.

(28) Complex particles which can be produced by the method of producing complex particles according to any one of the above (18) to (27).

(29) Complex particles comprising:

lead particles containing a lipid derivative or a fatty acid derivative of one or more substance(s) selected from sugars, peptides, nucleic acids and water-soluble polymers or a surfactant; and
a nucleic acid as a drug adhered to the lead particles.

(30) Complex particles comprising:

lead particles containing a lipid derivative or a fatty acid derivative of one or more substance(s) selected from sugars, peptides, nucleic acids and water-soluble polymers or a surfactant;
a drug adhered to the lead particles; and
an adhesion-competitive agent adhered to the lead particles.

(31) The complex particles according to the above (30), wherein the adhesion-competitive agent is one or more substance(s) selected from lipids, surfactants, nucleic acids, proteins, peptides and polymers.
(32) The complex particles according to the above (30), wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.
(33) The complex particles according to any one of the above (30) to (32), wherein the drug is a nucleic acid.
(34) The complex particles according to the above (29) or (33), wherein the nucleic acid as the drug is one or more substance(s) selected from genes, DNA, RNA, plasmids and siRNA.
(35) The complex particles according to any one of the above (29) to (34), wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.
(36) The complex particles according to any one of the above (29) to (34), wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.
(37) The complex particles according to any one of the above (29) to (36), wherein the lead particles are lead particles having electrostatic charge opposite to that of the drug.
(38) The complex particles according to any one of the above (29) to (36), wherein the lead particles are fine particles containing as a constituent component liposome containing a lipid with electrostatic charge opposite to that of the drug.

(39) A method of producing coated complex particles comprising the steps of:

preparing a liquid (liquid A) containing a polar organic solvent in which the complex particles according to any one of the above (28) to (38) are dispersed and a coating layer component is dissolved; and
coating the complex particles with a coating layer composed of the coating layer component by reducing the ratio of the polar organic solvent in the liquid A.

(40) The method of producing coated complex particles according to the above (39), wherein the step of preparing the liquid A comprises the steps of:

preparing a liquid (liquid B) containing a polar organic solvent in which the complex particles according to any one of the above (28) to (38) are dispersed;
preparing a liquid (liquid C) obtained by dissolving the coating layer component in a solvent containing a polar organic solvent which is the same as or different from that in the liquid B; and
mixing the liquid B and the liquid C.

(41) The method of producing coated complex particles according to the above (39) or (40), wherein the coating layer is a lipid membrane.
(42) The method of producing coated complex particles according to the above (41), wherein the coating layer is a coating layer containing a water-soluble polymer derivative.
(43) Coated complex particles which can be produced by the method of producing coated complex particles according to any one of the above (39) to (42).

(44) Coated complex particles comprising the complex particles according to any one of the above (28) to (38) and a coating layer for coating the complex particles, wherein in a solvent containing a polar solvent at a concentration within a range where the complex particles are not dissolved and can be dispersed therein, a coating layer component constituting the coating layer is dissolved when the concentration of the polar solvent is relatively high, and is deposited or assembled when the concentration of the polar solvent is relatively low.

(45) The coated complex particles according to the above (44), wherein the coating layer is a lipid membrane.

(46) The coated complex particles according to the above (45), wherein the coating layer is a coating layer containing a water-soluble polymer derivative.

(47) The coated complex particles according to any one of the above (44) to (46), wherein the average particles diameter of the coated complex particles are 300 nm or less.

Effect of the Invention

According to the present invention, a method of inhibiting aggregation of complex particles in which a drug is adhered to lead particles, a method of producing the complex particles and the like are provided. Further, a method of producing coated complex particles in which aggregation-inhibited complex particles are coated with a coating layer, coated complex particles that can be produced by the production method and the like are provided.

Best Mode for Carrying Out the Invention

Complex particles in the present invention mean particles which contains at least lead particles and a drug and in which the drug is adhered to the lead particles.

The above-mentioned drug (hereinafter referred to as drug A) is a drug adhered to lead particles in the complex particles in the present invention and preferably a drug electrostatically adhered to lead particles, and includes those electrostatically attracting a cation or an anion due to an electric charge in the molecule of the drug, intramolecular polarization or the like. Examples thereof include substances having a pharmacological activity such as a protein, a peptide, a nucleic acid, a low-molecular compound, a saccharide and a high-molecular compound and the like. Preferred examples include a nucleic acid, more preferred examples include a gene, DNA, RNA, an oligonucleotide (ODN), a plasmid and siRNA, and further more preferred examples include a plasmid and siRNA.

Examples of the protein or the peptide include bradykinin, angiotensin, oxytocin, vasopressin, adrenocorticotropin, calcitonin, insulin, glucagon, cholecystokinin, β-endorphin, a melanocyte inhibiting factor, a melanocyte stimulating hormone, a gastrin antagonist, neurotensin, somatostatin, brucine, cyclosporine, enkephalin, transferrin, an Arg-Gly-Asp (RGD) peptide, a thyroid hormone, a growth hormone, a gonadotropic hormone, a luteinizing hormone, asparaginase, arginase, uricase, carboxypeptidase, glutaminase, superoxide dismutase, a tissue plasminogen activator, streptokinase, interleukin, interferon, muramyl dipeptide, thymopoietin, a granulocyte colony stimulating factor, a granulocyte macrophage colony stimulating factor, erythropoietin, thrombopoietin, a trypsin inhibitor, lysozyme, an epidermal growth factor (EGF), an insulin-like growth factor, a nerve growth factor, a platelet-derived growth factor, a transforming growth factor, an endothelial cell growth factor, a fibroblast growth factor, a glial growth factor, thymosin and a specific antibody (such as an anti-EGF receptor antibody) and the like.

Examples of the nucleic acid include ODN such as an antisense oligonucleotide and a sense oligonucleotide, a gene, DNA, RNA, a plasmid, siRNA and the like. The nucleic acid includes derivatives in which an oxygen atom or the like contained in such as a phosphate moiety or an ester moiety in the nucleic acid structure has been substituted with another atom such as a sulfur atom. Incidentally, siRNA means a short double-stranded RNA.

Examples of the low-molecular compound include epsilon-aminocaproic acid, arginine hydrochloride, potassium L-aspartate, tranexamic acid, bleomycin sulfate, vincristine sulfate, cefazolin sodium, cephalothin sodium, citicoline, cytarabine, gentamicin sulfate, vancomycin hydrochloride, kanamycin sulfate, amikacin sulfate and the like.

Examples of the saccharide include sodium chondroitin sulfate, heparin sodium, dextran fluorescein and the like.

Examples of the high-molecular compound include sodium polyethylene sulfonate, a copolymer of divinyl ether with maleic anhydride (DIVEMA), a bonded product of a styrene-maleic anhydride copolymer with neocarzinostatin (SMANCS) and the like.

The lead particles in the present invention is fine particles containing as a constituent component, for example, a drug, lipid assembly, liposome, an emulsion particles, a polymer, a metal colloid, fine particles preparation or the like. Preferred examples include fine particles containing liposome as a constituent component. The lead particles in the present invention may contain as a constituent component a complex obtained by combining two or more of a drug, lipid assembly, liposome, an emulsion particles, a polymer, a metal colloid, fine particles preparation and the like, or may contain as a constituent component a complex obtained by combining a drug, lipid assembly, liposome, an emulsion particles, a polymer, a metal colloid, fine particles preparation or the like with another compound (such as a sugar, lipid or an inorganic compound).

The drug as a constituent component of the lead particles (hereinafter referred to as drug B) includes a drug which takes the form of particles in a solvent for dispersing the lead particles described below, a drug which forms a complex with the above-mentioned another compound and takes the form of particles in a solvent for dispersing the lead particles described below and the like. Examples thereof include lipid drug, a polymeric drug, a metal drug and the like, and specific examples include cisplatin, vitamin D, vitamin E, lentinan and the like.

The lipid assembly or the liposome is composed of, for example, lipid and/or a surfactant or the like. The lipid may be any of a simple lipid, a complex lipid and a derived lipid, and examples thereof include a phospholipid, a glyceroglycolipid, a sphingoglycolipid, a sphingoid, a sterol and the like, and preferred examples include a phospholipid. Further, examples of the lipid also include surfactants (the same definition as the surfactant described below), a polymer (the same definition as the polymer described below, specifically dextran, etc.), and lipid derivative such as a polyoxyethylene derivative (specifically, polyethylene glycol, etc.), and preferred examples include a polyethylene glycolated lipid. Examples of the surfactant include a nonionic surfactant, an anionic surfactant, a cationic surfactant, a zwitterionic surfactant and the like.

Examples of the phospholipid include natural and synthetic phospholipids such as phosphatidylcholine (specifically, soybean phosphatidylcholine, egg yolk phosphatidylcholine (EPC), distearoyl phosphatidylcholine, dipalmitoyl phosphatidylcholine, dimyristoyl phosphatidylcholine, dioleoyl phosphatidylcholine, etc.), phosphatidylethanolamine (specifically, distearoyl phosphatidylethanolamine (DSPE), dipalmitoyl phosphatidylethanolamine, dioleoyl phosphatidylethanolamine, etc.), glycerophospholipid (specifically, phosphatidylserine, phosphatidic acid, phosphatidylglycerol, phosphatidylinositol, lysophosphatidylcholine, etc.) sphingophospholipid (specifically sphingomyelin, ceramide phosphoethanolamine, ceramide phosphoglycerol, ceramide phosphoglycerophosphate, etc.) glycerophosphono lipid, sphingophosphonolipid, natural lecithin (specifically, egg yolk lecithin, soybean lecithin, etc.) and hydrogenated phospholipid (specifically hydrogenated phosphatidylcholine, etc.).

Examples of the glyceroglycolipid include sulfoxyribosyl glyceride, diglycosyl diglyceride, digalactosyl diglyceride, galactosyl diglyceride, glycosyl diglyceride and the like.

Examples of the sphingoglycolipid include galactosyl cerebroside, lactosyl cerebroside, ganglioside and the like.

Examples of the sphingoid include sphingan, icosasphingan, sphingosine, a derivative thereof and the like. Examples of the derivative thereof include those in which $-NH_2$ of sphingan, icosasphingan, sphingosine or the like is replaced with $-NHCO(CH_2)_xCH_3$ (in the formula, x represents an integer of 0 to 18, in particular, 6, 12 or 18 is preferred) and the like.

Examples of the sterol include cholesterol, dehydrocholesterol, lanosterol, β-sitosterol, campesterol, stigmasterol, brassicasterol, ergocasterol, fucosterol, 3β-[N-(N'N'-dimethylaminoethyl)carbamoyl cholesterol (DC-Chol) and the like.

Examples of the lipid other than these include N-[1-(2,3-dioleoylpropyl)]-N,N,N-trimethylammonium chloride (DOTAP), N-[1-(2,3-dioleoylpropyl)]-N,N- dimethylamine (DODAP), N-[1-(2,3-dioleyloxypropyl)- N,N,N-trimethylammonium chloride (DOTMA), 2,3- dioleyloxy-N-[2-(sperminecarboxyamido)ethyl]-N,N-dimethyl-1-propanaminium trifluoroacetate (DOSPA), N-[1-(2,3-ditetradecyloxypropyl)]-N,N-dimethyl-N-hydroxyethylammonium bromide (DMRIE), N-[1-(2,3-dioleyloxypropyl)]-N,N-dimethyl-N- hydroxyethylammonium bromide (DORIE) and the like.

Examples of the nonionic surfactants include polyoxyethylene sorbitan monooleate (specifically, Polysorbate 80, etc.), polyoxyethylene polyoxypropylene glycol (specifically, Pluronic F68, etc.), a sorbitan fatty acid (specifically, sorbitan monolaurate, sorbitan monooleate, etc.), a polyoxyethylene derivative (specifically, polyoxyethylene hydrogenated castor oil 60, polyoxyethylene lauryl alcohol, etc.), a glycerol fatty acid ester and the like.

Examples of the anionic surfactants include acylsarcosine, sodium alkylsulfate, alkylbenzene sulfonate, a sodium fatty acid having 7 to 22 carbon atoms and the like. Specific examples include sodium dodecyl sulfate, sodium lauryl sulfate, sodium cholate, sodium deoxycholate, sodium taurodeoxycholate and the like.

Examples of the cationic surfactants include an alkylamine salt, an acylamine salt, a quaternary ammonium salt, an amine derivative and the like. Specific examples include benzalkonium chloride, an acylaminoethyldiethylamine salt, an N-alkylpolyalkylpolyamine salt, a polyethylene polyamide fatty acid, cetyltrimethylammonium bromide, dodecyltrimethylammonium bromide, alkylpolyoxyethyleneamine, N-alkylaminopropylamine, a triethanolamine fatty acid ester and the like.

Examples of the zwitterionic surfactants include 3-[3-cholamidopropyl]dimethylammonio]-1-propane sulfonate, N-tetradecyl-N,N-dimethyl-3-ammonio-1- propane sulfonate and the like.

In the liposome, these lipid and surfactants are used alone or in combination, and preferably they are used in combination. As the combination in the case where they are used in combination, for example, a combination of two or more components selected from a hydrogenated soybean phosphatidylcholine, a polyethylene glycolated phospholipid and cholesterol, a combination of two or more components selected from distearoyl phosphatidylcholine, a polyethylene glycolated phospholipid and cholesterol, a combination of EPC and DOTAP, a combination of EPC, DOTAP and a polyethylene glycolated phospholipid, a combination of EPC, DOTAP, cholesterol and a polyethylene glycolated phospholipid, and the like can be exemplified.

Further, the liposome may contain a membrane stabilizer such as a sterol including cholesterol, an antioxidant such as tocopherol or the like as needed.

Examples of the lipid assembly include a spherical micelle, a spherical reversed micelle, a sausage-shaped micelle, a sausage-shaped reversed micelle, a plate-shaped micelle, a plate-shaped reversed micelle, hexagonal I, hexagonal II and an associated product comprising two or more lipid molecules.

Examples of the emulsion particles include oil-in-water (o/w) emulsion particles such as a fat emulsion, an emulsion composed of a nonionic surfactant and soybean oil, lipid emulsion and lipid nanosphere, water-in-oil-in-water (w/o/w) emulsion particles and the like.

Examples of the polymer include natural polymers such as albumin, dextran, chitosan, dextran sulfate and DNA, synthetic polymers such as poly-L-lysine, polyethyleneimine, polyaspartic acid, a copolymer of styrene with maleic acid; a copolymer of isopropylacrylamide with acrylpyrrolidone, PEG-modified dendrimer, polylactic acid, polylactic acid polyglycolic acid and polyethylene glycolated polylactic acid, a salt thereof and the like.

Here, the salt of the polymer includes, for example, a metal salt, an ammonium salt, an acid addition salt, an organic amine addition salt, an amino acid addition salt and the like. Examples of the metal salt include alkali metal salts such as a lithium salt, a sodium salt and a potassium salt, alkaline earth metal salts such as a magnesium salt and a calcium salt, an aluminum salt, a zinc salt and the like. Examples of the ammonium salt include salts of ammonium, tetramethylammonium and the like. Examples of the acid addition salt include inorganic acid salts such as a hydrochloric acid salt, a sulfuric acid salt, a nitric acid salt and a phosphoric acid salt, and organic acid salts such as an acetic acid salt, a maleic acid salt, a fumaric acid salt and a citric acid salt. Examples of the organic amine addition salt include addition salts of morpholine, piperidine and the like, and examples of the amino acid addition salt include addition salts of glycine, phenylalanine, aspartic acid, glutamic acid, lysine and the like.

Examples of the metal colloid include metal colloids including gold, silver, platinum, copper, rhodium, silica, calcium, aluminum, iron, indium, cadmium, barium, lead and the like.

Examples of the fine particles preparation include a microsphere, a microcapsule, a nanocrystal, lipid nanoparticles, a polymeric micelle and the like.

Preferably, the lead particles have electrostatic charge opposite to that of the drug A. Here, the electrostatic charge opposite to that of the drug A includes an electric charge, surface polarization and the like generating electrostatic attraction to an electric charge in the molecule in the drug, intramolecular polarization or the like. In order for the lead particles to have electrostatic charge opposite to that of the drug A, preferably the lead particles contains a charged substance having electrostatic charge opposite to that of the drug A, more preferably the lead particles contains lipid (a cationic lipid or an anionic lipid described below) having electrostatic charge opposite to that of the drug A.

The charged substance contained in the lead particles are classified into a cationic substance exhibiting a cationic property and an anionic substance exhibiting an anionic property. However, even if it is a zwitterionic substance having both cationic group and anionic group, the relative electronegativity changes depending on the pH, bonding to another substance or the like, it can be classified into a cationic substance or an anionic substance depending on the conditions. Such a charged substance may be used as a constituent component of the lead particles or may be used by adding it to the constituent component of the lead particles.

Examples of the cationic substance include the cationic substances among those illustrated in the above-mentioned definition of the lead particles (specifically, a cationic lipid, a cationic surfactants (the same definition as above), a cationic polymer and the like), a protein or a peptide with which a complex can be formed at a pH equal to or less than an isoelectric point, and the like.

Examples of the cationic lipid include DOTAP, DOTMA, DOSPA, DMRIE, DORIE, DC-Chol and the like.

Examples of the cationic polymer include poly-L-lysine, polyethyleneimine, polyfect, chitosan and the like.

The protein or the peptide with which a complex can be formed at a pH equal to or less than an isoelectric point is not particularly limited as long as it is a protein or a peptide with which a complex can be formed at a pH equal to or less than the isoelectric point of the substance. Examples thereof include albumin, orosomucoid, globulin, fibrinogen, pepsin, ribonuclease T1 and the like.

Examples of the anionic substance include the anionic substances among those illustrated in the above-mentioned definition of the lead particles (specifically, an anionic lipid, an anionic surfactants (the same definition as above), an anionic polymer and the like), a protein or a peptide, with which a complex can be formed at a pH equal to or greater than an isoelectric point, a nucleic acid and the like.

Examples of the anionic lipid include phosphatidylserine, phosphatidylglycerol, phosphatidylinositol, phosphatidic acid and the like.

Examples of the anionic polymer include polyaspartic acid, a copolymer of styrene with maleic acid, a copolymer of isopropylacrylamide with acrylpyrrolidone, PEG-modified dendrimer, polylactic acid, polylactic acid polyglycolic acid, polyethylene glycolated polylactic acid, dextran sulfate, sodium dextran sulfate, chondroitin sulfate, sodium chondroitin sulfate, hyaluronic acid, chondroitin, dermatan sulfate, heparan sulfate, heparin, ketaran sulfate, dextran fluorescein anionic and the like.

The protein or the peptide with which a complex can be formed at a pH equal to or greater than an isoelectric point is not particularly limited as long as it is a protein or a peptide with which a complex can be formed at a pH equal to or greater than the isoelectric point of the substance. Examples thereof include albumin, orosomucoid, globulin, fibrinogen, histone, protamine, ribonuclease, lysozyme and the like.

Examples of the nucleic acid as an anionic substance include DNA, RNA, a plasmid, siRNA, ODN and the like. It may have any length and any sequence as long as it does not exhibit a physiological activity.

Preferred examples of the lipid derivative or the fatty acid derivative of one or more substance(s) selected from sugars, peptides, nucleic acids and water-soluble polymers or the surfactant contained in the lead particles in the present invention include a glycolipid or lipid derivative or a fatty acid derivative of a water-soluble polymer. Specific examples include a polyethylene glycolated lipid, a polyethylene glycol sorbitan fatty acid ester, a polyethylene glycol fatty acid ester, a polyglycerolated lipid, a polyglycerol fatty acid ester, polyoxyethylene polyoxypropylene glycol, a glycerol fatty acid ester, a polyethylene glycol alkyl ether and the like. More preferred examples include lipid derivative or a fatty acid derivative of a water-soluble polymer. The lipid derivative or the fatty acid derivative of one or more substance(s) selected from sugars, peptides, nucleic acids and water-soluble polymers or the surfactant in the present invention is preferably a substance having a dual character that a part of the molecule has a property of binding to a constituent component of the lead particles due to, for example, hydrophobic affinity, electrostatic force or the like, and other part has a property of binding to a solvent used in the production of the lead particles due to, for example, hydrophilic affinity, electrostatic force or the like. Hereinafter, the lipid derivative or the fatty acid derivative of one or more substance(s) selected from sugars, peptides, nucleic acids and water-soluble polymers or the surfactant is represented by an aggregation-inhibiting substance.

Examples of the lipid derivative or the fatty acid derivative of one or more substance(s) selected from sugars, peptides and nucleic acids include those comprising a sugar such as sucrose, sorbitol or lactose, a peptide such as a casein-derived peptide, an egg white-derived peptide, a soybean-derived peptide or glutathione, a nucleic acid such as DNA, RNA, a plasmid, siRNA or ODN, or the like and any of the lipid illustrated in the above-mentioned definition of the lead particles or a fatty acid such as stearic acid, palmitic acid or lauric acid bonded to each other and the like.

Examples of the lipid derivative or the fatty acid derivative of a sugar include the glyceroglycolipid and the sphingoglycolipid illustrated in the above-mentioned definition of the lead particles and the like.

Examples of the lipid derivative or the fatty acid derivative of a water-soluble polymer include those comprising polyethylene glycol, polyethyleneimine, polyvinyl alcohol, polyacrylic acid, polyacrylamide, oligosaccharide, dextrin, a water-soluble cellulose, dextran, chondroitin sulfate, polyglycerol, chitosan, polyvinylpyrrolidone, polyaspartate amide, poly-L-lysine, mannan, pullulan, oligoglycerol or the like or a derivative thereof and any of the lipid illustrated in the above-mentioned definition of the lead particles or a fatty acid such as stearic acid, palmitic acid, myristic acid or lauric acid bonded to each other and the like. More preferably, lipid derivative or a fatty acid derivative of a polyethylene glycol derivative or a polyglycerol derivative can be exemplified, and further more preferably, lipid derivative or a fatty acid derivative of a polyethylene glycol derivative can be excemplifed.

Examples of the lipid derivative or the fatty acid derivative of a polyethylene glycol derivative include a polyethylene glycolated lipid [specifically, polyethylene glycol phosphatidyl ethanolamine (more specifically, 1,2-distearoyl-sn-glycero-3- phosphoethanolamine-N-[methoxy(polyethylene glycol)- 2000] (PEG-DSPE) and the like), polyoxyethylene hydrogenated castor oil 60, Cremophor EL and the like], a polyethylene glycol sorbitan fatty acid ester (specifically, polyoxyethylene sorbitan monooleate and the like), a polyethylene glycol fatty acid ester and the like, and more preferred examples include a polyethylene glycolated lipid.

Examples of the lipid derivative or the fatty acid derivative of a polyglycerol derivative include a polyglycerolated lipid (specifically, polyglycerol phosphatidyl ethanolamine and the like), a polyglycerol fatty acid ester and the like, and more preferred examples include a polyglycerolated lipid.

Examples of the surfactant include the surfactant illustrated in the above-mentioned definition of the lead particles, a polyethylene glycol alkyl ether and the like, and preferred examples include polyoxyethylene polypropylene glycol, a glycerol fatty acid ester, a polyethylene glycol alkyl ether and the like.

As the adhesion-competitive agent in the present invention, for example, a substance having the same electrostatic charge as that of the drug A and the like can be exemplified, and a substance electrostatically adhered to the lead particles due to the electrostatic attraction to a cation or an anion by an electric charge in the molecule, intramolecular polarization or the like is included. Examples thereof include a lipid, surfactants, a nucleic acid, a protein; a peptide, a polymer and the like. Examples of the lipid, the surfactant, the nucleic acid, the protein, the peptide and the polymer include the cationic lipids, the anionic lipids, the cationic surfactants, the anionic surfactants, the nucleic acids, the proteins, the peptides, the cationic polymers and the anionic polymers illustrated in the above-mentioned definition of the charged substance and the like. Preferred examples include the cationic polymers and the anionic polymers illustrated in the above-mentioned definition of the charged substance and the like, and more preferred examples include one or more substance(s) selected from dextran, sulfate, sodium dextran sulfate, chondroitin sulfate, sodium chondroitin sulfate, hyaluronic acid, chondroitin, dermatan sulfate, heparan sulfate, heparin, ketaran sulfate, dextran fluorescein anionic, poly-L-lysine, polyethyleneimine, polyfect, chitosan and the like. The adhesion-competitive agent preferably was electrostatically adhered to the lead particles, and is preferably a substance with a size which does not allow the crosslinking formation to aggregate the lead particles even if the substance is adhered to the lead particles, or a substance having a moiety in its molecule, which repels the adhesion of the lead particles thereby inhibiting the aggregation of the lead particles. Further, particularly in the case where the drug A is a large drug with a molecular weight of 5000 or more (for example, a gene, DNA, RNA, a plasmid, siRNA or the like), to further attach the adhesion-competitive agent to the lead particles are one of the most preferred embodiments of the present invention.

The aggregation inhibitor for the present invention contains the aggregation-inhibiting substance in the present invention and may contain any other substance as long as the substance does not inhibit the aggregation-inhibiting action of the aggregation-inhibiting substance.

Inhibition of aggregation of complex particles in which a drug is adhered to lead particles in the present invention can be carried out by incorporating the aggregation-inhibiting substance in the lead particles. Specifically, for example, it is performed by dispersing or dissolving the drug A so as to be contained in a liquid in which the lead particles containing the aggregation-inhibiting substance are dispersed and allowing the drug adhered to the lead particles, and aggregation of the complex particles during the production of the complex particles and/or aggregation of the complex particles after the production are/is inhibited. Further, preferably, when the drug A is dispersed or dissolved so as to be contained in the liquid, by further incorporating the adhesion-competitive agent in the liquid and allowing the adhesion-competitive agent adhered to the lead particles along with the drug, aggregation of the complex particles are further inhibited.

More specifically, the method of inhibiting aggregation of the present invention can be carried out in a method of producing complex particles in which a drug is adhered to lead particles, which comprises, for example, the steps of preparing a liquid in which the lead particles containing the aggregation-inhibiting substance are dispersed, and dispersing or dissolving the drug A so as to be contained in the liquid in which the lead particles are dispersed (for example, the step of dispersing or dissolving the drug A by adding it to the liquid in which the lead particles are dispersed, the step of adding a liquid in which the drug A is dispersed or dissolved to the liquid in which the lead particles are dispersed, or the like). Here, specific examples of the complex particles obtained by the step of dispersing or dissolving the drug A so as to be contained in the liquid in which the lead particles are dispersed include complex particles formed by dispersing or dissolving a nucleic acid as a drug so as to be contained in a liquid in which fine particles containing as a constituent component liposome containing a cationic lipid are dispersed and allowing the nucleic acid as a drug adhered to the fine particles containing as a constituent component liposome containing a cationic lipid, in a similar manner; complex particles formed by allowing a nucleic acid as a drug adhered to fine particles containing as a constituent component lipid assembly containing a cationic lipid, complex particles in which a nucleic acid as a drug is adhered to fine particles containing as a constituent component a polymer containing a cationic polymer such as poly-L-lysine, complex particles in which a protein is adhered to fine particles containing as a constituent component liposome or lipid assembly containing an anionic lipid such as phosphatidic acid, complex particles in which a protein is adhered to fine particles containing as a constituent component a polymer containing an anionic polymer such as styrene-maleic acid, complex particles in which a protein is adhered to fine particles containing as a constituent component a polymer containing a cationic polymer such as poly-L-lysine, complex particles in which a protein is adhered to fine particles containing as a constituent component liposome or lipid assembly containing a cationic lipid and the like. Further, the step of dispersing or dissolving the drug A so as to be contained in the liquid in which the lead particles are dispersed is preferably a step of further incorporating the adhesion-competitive agent in the liquid in which the drug A is dispersed or dissolved and adding the liquid to the liquid in which the lead particles are dispersed. In this case, both the drug A and the adhesion-competitive agent is adhered to the lead particles to form complex particles, and aggregation of the complex particles during the production of the complex particles and aggregation of the complex particles after the production are further inhibited.

The lead particles containing the aggregation-inhibiting substance can be produced by or in accordance with a known production method, and may be produced by any production method as long as the aggregation-inhibiting substance is incorporated in the lead particles. For example, in the production of fine particles containing as a constituent component liposome containing the aggregation-inhibiting substance, which is one of the lead particles, a known liposome preparation method can be applied. As the known liposome preparation method, for example, liposome preparation method by Bangham, et al. [see "Journal of Molecular Biology" (J. Mol. Biol.), vol. 13, pp. 238-252 (1965)], an ethanol injection method [see "Journal of Cell Biology" (J. Cell Biol.), vol. 66, pp. 621-634 (1975)], a French press method [see "FEBS Letters" (FEBS Lett.), vol. 99, pp. 210-214 (1979)], a freeze-thaw method [see "Archives of Biochemistry and Biophysics" (Arch. Biochem. Biophys.), vol. 212, pp. 186-194 (1981)], a reverse phase evaporation method [see "Proceedings of the National Academy of Science United States of America" (Proc. Natl. Acad. Sci. USA), vol. 75, pp. 4194-4198 (1978)], a pH gradient method (see, for example, Japanese Patent No. 2,572,554, Japanese Patent No. 2,659,136, etc.) and the like. As a solution for suspending liposome in the production of the liposome, for example, water, an acid, an alkali, any of various buffers, a physiological saline solution, an amino acid infusion or the like can be used. Further, in the production of the liposome, it is also possible to add an antioxidant such as citric acid, ascorbic acid, cysteine or ethylenediamine tetraacetic acid (EDTA), a isoosmotic agent such as glycerol, glucose, sodium chloride or the like. Further, the liposome can be prepared by dissolving lipid or the like in, for example, an organic solvent such as ethanol, distilling off the solvent, adding a physiological saline solution or the like and stirring the mixture by shaking, thereby forming liposome.

Further, surface improvement of the liposome can be optionally carried out using, for example, a nonionic surfactants (the same definition as above), a cationic surfactants (the same definition as above), an anionic surfactants (the same definition as above), a polymer, a polyoxyethylene derivative or the like, and such a surface-improving liposome is also used as a constituent component of the lead particles in the present invention [see "Stealth Liposome", edited by D. D. Lasic and F. Martin, CRC Press Inc., USA, pp. 93-102 (1995)]. Examples of the polymer include dextran, pullulan, mannan, amylopectin, hydroxyethylstarch and the like. Examples of the polyoxyethylene derivative include Polysorbate 80, Pluronic F68, polyoxyethylene hydrogenated castor oil 60, polyoxyethylene lauryl alcohol, PEG-DSPE and the like. The surface improvement of the liposome can be employed as one of the methods of incorporating lipid derivative or a fatty acid derivative of one or more substance(s) selected from sugars, peptides, nucleic acids and water-soluble polymers or a surfactant in the lead particles.

An average particles diameter of the liposome can be freely selected upon demand. Examples of a method of adjusting the average particles diameter include an extrusion method and a method in which a large multilamellar liposome vesicle (MLV) is mechanically pulverized (specifically using Manton-gaulin, a microfluidizer or the like) [see "Emulsion and Nanosuspensions for the Formulation of Poorly Soluble Drugs", edited by R. H. Muller, S. Benita and B. Bohm, Scientific Publishers, Stuttgart, Germany, pp. 267-294 (1998)] and the like.

In addition, the method of producing a complex obtained by combining two or more substances selected from, for example, the drug B, lipid assembly, liposome, an emulsion particles, a polymer, a metal colloid, fine particles preparation and the like, which constitute the lead particles may be, for example, a production method in which the drug B is only mixed with a lipid, a polymer or the like in water. At this time, a granulation step, a sterilization step or the like can be further added as needed. Further, it is also possible to perform the formation of the complex in any of various solvents such as acetone and an ether.

The ratio of the aggregation-inhibiting substance to the total lead particles in the method of inhibiting aggregation of complex particles in which a drug is adhered to the lead particles of the present invention is preferably 1:0.9 to 1:0.01, more preferably 1:0.7 to 1:0.1, further more preferably 1:0.6 to 1:0.2, the most preferably 1:0.5 to 1:0.3 in ratio by weight.

As for the size of the lead particles, an average particles diameter is preferably 10 nm to 300 nm, more preferably 50 nm to 150 nm, further more preferably 50 nm to 100 nm.

A solvent in which the lead particles are dispersed is a solvent in which the lead particles are not dissolved, and is preferably a solvent that does not inhibit the drug A from adhering to the lead particles in the step of producing the complex particles. Examples of the solvent in which the lead particles are dispersed include a solvent containing water or the like, and preferred examples include water. On the other hand, the lead particles are preferably lead particles which are dispersed in water or the like. In the case where the solvent used in the production of the lead particles are water, it is possible to produce the complex particles in the same liquid successively following the production of the lead particles.

In the step of dispersing or dissolving the drug A or the drug A and the adhesion-competitive agent so as to be contained in a liquid in which the lead particles containing the aggregation-inhibiting substance are dispersed, when a liquid in which the drug A or the drug A and the adhesion-competitive agent are dispersed or dissolved is added to the liquid in which the lead particles are dispersed, a solvent to be used for the liquid in which the drug A or the drug A and the adhesion-competitive agent is/are dispersed or dissolved may be any as long as it is a solvent which does not inhibit the drug A from adhering to the lead particles in a liquid mixture after mixing the liquid in which the lead particles are dispersed with the liquid in which the drug A or the drug A and the adhesion-competitive agent are dispersed or dissolved. Examples of the solvent in which the drug A or the drug A and the adhesion-competitive agent are dispersed or dissolved include a solvent containing water or the like, and preferred examples include water. On the other hand, the drug A and the adhesion-competitive agent are preferably a drug A and an adhesion-competitive agent that are dissolved or dispersed in water or the like, respectively, and more preferred are a drug A and an adhesion-competitive agent that are dissolved in water, respectively.

The ratio of the lead particles to the liquid in which the lead particles are dispersed is not particularly limited as long as the drug A or the drug A and the adhesion-competitive agent can be adhered to the lead particles, however, it is preferably 1 $\mu$g/mL to 1 g/mL, more preferably 0.1 to 500 mg/mL. Further, in the step of dispersing or dissolving the drug A or the drug A and the adhesion-competitive agent so as to be contained in the liquid in which the lead particles containing the aggregation-inhibiting substance are dispersed, when a liquid in which the drug A or the drug A and the adhesion-competitive agent is/are dispersed or dissolved is added to the liquid in which the lead particles are dispersed, the ratio of the total amount of the drug A and the adhesion-competitive agent to the liquid in which the drug A or the drug A and the adhesion-competitive agent is/are dispersed or dissolved is not particularly limited as long as the drug A or the drug A and the adhesion-competitive agent can be adhered to the lead particles, however, it is preferably 1

μg/mL to 1 g/mL, more preferably 0.1 to 400 mg/mL. The ratio of the total amount of the drug A and the adhesion-competitive agent to the lead particles are preferably 1:1 to 1000:1, more preferably 2:1 to 200:1 in ratio by weight.

The complex particles of the present invention is complex particles comprising lead particles containing an aggregation-inhibiting substance and a nucleic acid as a drug adhered to the lead particles, or a drug adhered to the lead particles and an adhesion-competitive agent adhered to the lead particles. The definition of each constituent component in the complex particles of the present invention is the same as each definition described above.

The method of producing complex particles of the present invention is a production method comprising the step of allowing a nucleic acid as a drug or a drug and an adhesion-competitive agent adhered to lead particles by dispersing or dissolving the nucleic acid or the drug and the adhesion-competitive agent so as to be contained in a liquid in which the lead particles containing an aggregation-inhibiting substance are dispersed. The production method comprising the step of allowing a nucleic acid as a drug adhered to lead particles by dispersing or dissolving the nucleic acid so as to be contained in a liquid in which the lead particles containing an aggregation-inhibiting substance are dispersed can be carried out by the same method as illustrated in the above-mentioned description of the method of inhibiting aggregation of complex particles in which the drug is adhered to the lead particles of the present invention using the nucleic acid as the drug. The production method comprising the step of allowing a drug and an adhesion-competitive agent adhered to lead particles by dispersing or dissolving the drug and the adhesion-competitive agent so as to be contained in a liquid in which the lead particles containing an aggregation-inhibiting substance are dispersed can be carried out by the same method as illustrated in the description in the case where an adhesion-competitive agent is used in the above-mentioned description of the method of inhibiting aggregation of complex particles in which the drug is adhered to the lead particles of the present invention.

As for the size of the complex particles in the present invention and the complex particles of the present invention, an average particles diameter is preferably 50 nm to 300 nm, more preferably 50 nm to 200 nm, further more preferably 50 nm to 150 nm.

Further, following the step of producing the complex particles in the present invention and the complex particles of the present invention, by adding a charged substance or a liquid in which a charged substance is dispersed or dissolved to allow the charged substance adhered to the complex particles, a multicomplex particles can also be obtained. For example, it is possible to form a multicomplex particles by preparing fine particles containing as a constituent component liposome, which is lead particles, using a cationic substance and an aggregation-inhibiting substance in water, then adding, for example, a nucleic acid as the drug A (preferably along with an adhesion-competitive agent), and further adding, for example, an anionic substance. In addition, the complex particles in the present invention and the complex particles of the present invention can be formed into coated complex particles.

The coated complex particles of the present invention is coated complex particles comprising at least the complex particles of the present invention and a coating layer for coating the complex particles, and examples thereof include coated complex particles in which, in a solvent containing a polar solvent at a concentration within a range where the complex particles are not dissolved and can be dispersed therein, a coating layer component constituting the coating layer is dissolved when the concentration of the polar solvent is relatively high, and is deposited or assembled when the concentration of the polar solvent is relatively low and the like.

Examples of the coating layer component constituting the coating layer in the coated complex particles of the present invention include the lipids, the surfactant and the polymers illustrated in the above-mentioned definition of the lead particles and the like, preferred examples include one or more substance(s) selected from the lipid and the surfactant illustrated in the above-mentioned definition of the lead particles, more preferred examples include one or more substances selected from lipid and surfactants, which will make a lipid membrane to be the coating layer, and further more preferred examples include a phospholipid.

Further, examples of the lipid to be used in the case where the coating layer is a lipid membrane include a synthetic lipid and the like. Examples of the synthetic lipid include fluorinated phosphatidylcholine, a fluorinated surfactants, dialkylammonium bromide and the like. These may be used alone or in combination with another lipid or the like. Further, in the case where the coating layer is a lipid membrane, the coating layer preferably contains a water-soluble polymer derivative. Examples of the water-soluble polymer derivative include the lipid derivatives or the fatty acid derivatives of a water-soluble polymer illustrated in the above-mentioned definition of the aggregation-inhibiting substance and the like, and preferred examples include the polyethylene glycolated phospholipids illustrated in the above-mentioned definition of the aggregation-inhibiting substance and the like. Further, the water-soluble polymer derivative is preferably a substance having a dual character that a part of the molecule has a property of binding to the aggregation-inhibiting substance or the adhesion-competitive agent in the present invention due to, for example, hydrophilic affinity, electrostatic force or the like, and other part has a property of binding to other coating layer components due to, for example, hydrophobic affinity, electrostatic force or the like. By using such a substance, the efficiency of the coating of the complex particles of the present invention is increased. The ratio of the water-soluble polymer derivative to the total coating layer components is preferably 1:0.5 to 1:0.01, more preferably 1:0.25 to 1:0.01, further more preferably 1:0.15 to 1:0.02 in ratio by weight.

The coated complex particles of the present invention can be produced, for example, by a production method comprising the steps of preparing a liquid (liquid A) containing a polar organic solvent in which the complex particles of the present invention are dispersed and a coating layer component is dissolved, and coating the complex particles with a coating layer by reducing the ratio of the polar organic solvent in the liquid A. In this case, the coated complex particles are obtained in the form of a suspension (liquid D). The solvent in the liquid A is a solvent in which the complex particles are not dissolved and the coating layer component is dissolved. In the liquid D in which the ratio of the polar organic solvent in the liquid A is reduced, the complex particles are not dissolved and the coating layer component is not dissolved or is assembled. In the case where the solvent in the liquid A is a polar organic solvent alone, for example, by adding a solvent (liquid E) containing a solvent other than a polar organic solvent mixable with the polar organic solvent preferably gradually, the ratio of the polar organic solvent can be reduced relatively. Here, the liquid E is a solvent containing a solvent other than a polar organic solvent and may contain a polar organic solvent. Further, in the case where the solvent in the liquid A is a liquid mixture of a polar organic solvent and a solvent other than a polar organic solvent, for example, by adding a solvent (liquid F) containing a solvent other than a polar organic solvent mixable with the polar organic solvent, and/or selectively removing the polar organic solvent by distillation by evaporation, semipermeable membrane separation, fractional distillation or the like, the ratio of the polar organic solvent can be reduced. Here, the liquid F is a solvent containing a solvent other than a polar organic solvent, and may also contain a polar organic solvent as long as the ratio of the polar organic solvent is lower than that in the liquid A. Examples of the polar organic solvent include alcohols such as methanol, ethanol, n-propanol, 2-propanol, n-butanol, 2-butanol, and tert-butanol, glycols such as glycerol, ethylene glycol and propylene glycol, polyalkylene glycols such as polyethylene glycol and the like, and preferred examples include ethanol. Examples of the solvent other than a polar organic solvent include water, liquid carbon dioxide, a liquid hydrocarbon, a halogenated carbon, a halogenated hydrocarbon and the like, and preferred examples include water. Further, the liquid A, the liquid E and the liquid F may contain an ion, a buffer component or the like.

The combination of a polar organic solvent with a solvent other than a polar organic solvent is preferably a combination of solvents that are mixable with each other and can be selected by considering the solubility of the above-mentioned complex particles and the above-mentioned coating layer component in the solvents in the liquid A and the liquid D, and the liquid E and the liquid F. On the other hand, the above-mentioned complex particles preferably has a low solubility in any of the solvents in the liquid A and the liquid D, and the liquid E and the liquid F, and also preferably has a low solubility in any of a polar organic solvent and a solvent other than a polar organic solvent. The coating layer component preferably has a low solubility in the solvent in the liquid D and the liquid F, and preferably has a high solubility in the solvent in the liquid A and the liquid E, and preferably has a high solubility in a polar organic solvent and preferably has a low solubility in a solvent other than a polar organic solvent. Here, the complex particles having a low solubility means that the dissolubility of each component contained in the complex particles such as the lead particles, the drug A or the adhesion-competitive agent in the solvent is low, and even if the respective solubility of the components are high, it is sufficient if the dissolubility of each component became low due to the binding or the like between the respective components. For example, even in the case where the solubility of any of the components contained in the lead particles in the solvent in the liquid A is high, if the solubility of the adhesion-competitive agent in the solvent in the liquid A is low, the elution of the other components in the complex particles are inhibited, whereby the solubility of the complex particles in the solvent in the liquid A can be lowered. That is, in the case where an adhesion-competitive agent with a lower solubility in the solvent in the liquid A than the solubility of any of the other components in the complex particles in the solvent in the liquid A is selectively used, the adhesion-competitive agent inhibits the elution of the other components of the complex particles in the production of the coated complex particles and has an effect on improving the productivity and yield.

The ratio of the polar organic solvent in the liquid A is not particularly limited as long as it satisfies the requirements that the complex particles of the present invention is present therein without being dissolved and the coating layer component for coating the complex particles are dissolved therein, and varies depending on the solvent or the complex particles to be used, the type of coating layer component or the like. However, it is preferably 30 % by volume or more, more preferably 60 to 90% by volume. Further, the ratio of the polar organic solvent in the liquid D is not particularly limited as long as it allows the coating layer component to form the coating layer on the surface of the complex particles of the present invention, however, it is preferably 50% by volume or less.

The step of preparing the liquid A may be a step of preparing the liquid A by adding the above polar organic solvent, the above complex particles and the above coating layer component, or the above polar organic solvent, the above complex particles, the above coating layer component and the solvent other than the above polar organic solvent in any order as long as the complex particles are not dissolved. Preferably, a step of preparing the liquid A by preparing a liquid (liquid B) containing a polar organic solvent in which the complex particles of the present invention are dispersed, preparing a liquid (liquid C) in which a coating layer component is dissolved in a solvent containing a polar organic solvent that is the same as or different from the polar organic solvent in the liquid B and mixing the liquid B and the liquid C can be exemplified. When the liquid A is prepared by mixing the liquid B and the liquid C, it is preferred to mix them gradually.

As a preferred method of producing coated complex particles of the present invention in which the coating layer is a lipid membrane, for example, the following method can be exemplified.

(Step 1) The complex particles of the present invention are dispersed (suspended) in an aqueous solution containing a polar organic solvent, preferably in an aqueous solution containing an alcohol such as ethanol.

(Step 2) Lipid which will be lipid membrane and/or a surfactant (a component constituting the lipid membrane) are/is dissolved in an aqueous solution containing a polar organic solvent which is the same as or different from the above-mentioned aqueous solution containing a polar organic solvent, preferably in the same aqueous solution containing a polar organic solvent or in a polar organic solvent. At this time, a water-soluble polymer derivative (such as a PEG-modified lipid derivative) may be further added thereto, and the amount of the water-soluble polymer derivative to be added here is not particularly limited.

(Step 3) The liquid obtained in the step 1 and the liquid obtained in the step 2 are mixed.

(Step 4) Water is added little by little to the liquid mixture prepared in the step 3, or dialysis of the liquid mixture is carried out, or the polar organic solvent is distilled off from the liquid mixture so as to reduce the relative ratio of the polar organic solvent in the liquid mixture, whereby coated complex particles coated with lipid membrane is obtained in the form of a suspension.

The coated complex particles of the present invention can be basically produced by a similar method to the above method regardless of the type of complex particles to be used or the type of coating layer component to be used. Coated complex particles in which the lead particles are fine particles containing as a constituent component liposome, the coating layer component is lipid and/or a surfactant and the coating layer is a lipid membrane is classified into liposome in a narrow sense based on its structure. Coated complex particles in which the lead particles are other than fine particles containing as a constituent component liposome, the coating layer component is lipid and/or a surfactant and the coating layer is a lipid membrane is classified into liposome in a wide sense. In the present invention, it is more preferred that both the constituent component of the lead particles and the coated complex particles are liposome.

The ratio of the complex particles of the present invention to be used in the method of producing coated complex particles of the present invention to the liquid A and the liquid B is not particularly limited as long as it allows the complex particles to be coated with the coating layer component, however, it is preferably 1 $\mu$g/mL to 1 g/mL, more preferably 0.1 to 500 mg/mL. Further, the ratio of the coating layer component (such as a lipid) to be used to the liquid A and the liquid C is not particularly limited as long as it allows the complex particles of the present invention to be coated, however, it is preferably 1 $\mu$g/mL to 1 g/mL, more preferably 0.1 to 400 mg/mL. The ratio of the coating layer component to the complex particles of the present invention is preferably 1:0.1 to 1:1000, more preferably 1:1 to 1:10 in ratio by weight.

Further, as for the size of the coated complex particles of the present invention and the coated complex particles obtained by the method of producing coated complex particles of the present invention, an average particles diameter is preferably 350 nm or less, more preferably 300 nm or less, further more preferably 200 nm or less. Specifically, for example, an injectable size is preferred.

Further, the coated complex particles obtained above can be modified with a substance such as a protein including an antibody and the like, a saccharide, a glycolipid, an amino acid, a nucleic acid or any of various low-molecular compounds and high-molecular compounds, and such coated complex particles obtained by modification is included in the coated complex particles of the present invention. For example, in order to apply to targeting, it is possible that the coated complex particles obtained above is further subjected to a surface modification of the lipid membrane using a protein such as an antibody, a peptide, a fatty acid or the like [see "Stealth Liposome", edited by D. D. Lasic and F. Martin, CRC Press Inc., USA, pp. 93-102, (1995)]. Further, in the same manner as in the case of liposome which is a constituent component of the lead particles, surface improvement can also be optionally carried out using, for example, a nonionic surfactants (the same definition as above), a cationic surfactants (the same definition as above), an anionic surfactants (the same definition as above), a polymer (the same definition as above), a polyoxyethylene derivative (the same definition as above) or the like, and such coated complex particles subjected to the surface modification of the lipid membrane or the surface improvement is also included in the coated complex particles of the present invention.

The coated complex particles of the present invention can be used, for example, as a preparation intended for stabilization of a drug in a living body component such as a blood component, gastrointestinal juice or the like, reduction of side effects, increase in the accumulation property of a drug in a target organ such as a tumor, improvement in absorption of a drug orally or via mucous membrane or the like.

In the case where the coated complex particles of the present invention is used as a preparation, it is also possible to use the suspension of the coated complex particles prepared by the method described above as it is in the form of, for example, an injection or the like. However, it can also be used after removing the solvent from the suspension by, for example, filtration, centrifugation or the like, or after lyophilizing the suspension or the suspension supplemented with diluent such as mannitol, lactose, trehalose, maltose or glycine.

In the case of an injection, it is preferred that an injection is prepared by mixing, for example, water, an acid, an alkali, any of various buffers, a physiological saline solution, an amino acid infusion or the like with the suspension of the coated complex particles or the coated complex particles obtained by removing the solvent or lyophilization. Further, it is possible to prepare an injection by adding an antioxidant such as citric acid, ascorbic acid, cysteine or EDTA, an isotonic agent such as glycerol, glucose or sodium chloride or the like. Further, it can also be cryopreserved by adding a cryopreservation agent such as glycerol.

Further, the coated complex particles of the present invention may be formulated into an oral preparation such as a capsule, a tablet or a granule by granulating along with an appropriate excipient or the like, drying or the like.

Hereinafter, by way of Examples, the present invention will be described specifically. However, the present invention is not limited to these Examples.

Incidentally, ODN used in the Examples is a phosphorothioate-type, 5'-end FITC-labeled 20-mer, 5'ACTAGTGGCTAGCGAATCTC3', available from Takara Bio Inc.

Further, the plasmid used in the Examples is a 8.5-kb plasmid containing a β-galactosidase gene linked to CAG promoter (hereinafter abbreviated as pCAG-LacZ) or a 6.1-kb plasmid containing a RLuc gene linked to CAG promoter (hereinafter abbreviated as pCAG-RLuc).

The pCAG-Rluc plasmid was prepared by the following method.

Plasmid pRL-null vector (1 μg) (manufactured by Promega) was dissolve in 30 μL of a buffer (pH 7.5) [a buffer (pH 7.5) means universal buffer H (50 mmol/L Tris-hydroxymethyl aminomethane hydrochloride, 6.6 mmol/L magnesium chloride, 10 mmol/L dithiothreitol and 100 mmol/L sodium chloride manufactured by Takara Shuzo) and the same applies hereinafter], 10 units of restriction enzymes SalI and EcoRI were added thereto and a digestion reaction was carried out at 37°C for 2 hours. The obtained reaction solution was subjected to agarose gel electrophoresis, and a 3.3-kbp DNA fragment was recovered using a purification kit [a purification kit means QIAEX II Gel Extraction Kit (manufactured by QIAGEN) and the same applies hereinafter].

Then, 1 μg of plasmid pBSKS(+)CAG promoter described in International Publication WO 01/33957 was dissolved in 30 μL of a buffer (pH 7.5), restriction enzymes SalI and EcoRI were added thereto and a digestion reaction was carried out at 37°C for 2 hours. The obtained reaction solution was subjected to agarose gel electrophoresis, and a 1.7-kbp DNA fragment containing CAG promoter was recovered using a purification kit.

The thus obtained 3.3-kbp SalI-EcoRI fragment (0.1 μg) derived from plasmid pRL-null vector and the thus obtained 1.7-kbp SalI-EcoRI fragment (0.1 μg) derived from plasmid pBSKS(+)CAG promoter were dissolved in 30 μL of T4 ligase buffer [66 mmol/L Tris-hydroxymethyl aminomethane hydrochloride, 10 mmol/L magnesium chloride, 1 mmol/L dithiothreitol and 0.1 mmol of adenosine triphosphate, manufactured by Takara Shuzo], 100 units of T4 DNA ligase (manufactured by Takara Shuzo) was added thereto, and a ligation reaction was carried out at 16°C for 16 hours.

By using the obtained reaction solution, *E. coli DH5α* (manufactured by Toyobo Co.) was transformed in accordance with the method by Cohen et al. ["see "Proceedings of the National Academy of Science United States of America" (Proc. Natl. Acad. Sci. USA), vol. 69, pp. 2110-2114 (1972)], whereby an ampicillin resistant strain was obtained. In accordance with a known method, pCAG-Rluc plasmid was isolated from the transformant.

Further, siRNA used in the Examples is siRNA comprising a 5'-end FITC-labeled sense sequence: 5'CUGGAUCGUAAGAAGGCAGdTdT3' and an antisense sequence: 5'CUGCCUUCUUACGAUCCAGdTdT3'.

Example 1

DOTAP (manufactured by Avanti, the same applies hereinafter), PEG-DSPE (manufactured by NOF Corporation, the same applies hereinafter) and distilled water were mixed such that the ratio of DOTAP/PEG-DSPE/distilled water was 30 mg/6 mg/mL, and the mixture was stirred by shaking with a vortex mixer. The obtained suspension was passed, at room temperature, through a polycarbonate membrane filter of 0.4 μm (pore size) (manufactured by Whatman, the same applies hereinafter) for 4 times and through a polycarbonate membrane filter of 0.1 μm pore size (manufactured by Whatman, the same applies hereinafter) for 10 times and then through a polycarbonate membrane filter of 0.05 μm pore size (manufactured by Whatman, the same applies hereinafter) for 24 times, whereby lead particles were prepared. To 0.02 mL of the obtained suspension of lead particles, 0.01 mL of a 15 mg/mL aqueous solution of ODN was added, whereby complex particles were prepared.

Example 2

DOTAP, PEG-DSPE and distilled water were mixed such that the ratio of DOTAP/PEG-DSPE/distilled water was 30 mg/9 mg/mL, and the mixture was stirred by shaking with a vortex mixer. The obtained suspension was passed, at room temperature, through a polycarbonate membrane filter of 0.4 μm pore size for 4 times and through a polycarbonate membrane filter of 0.1 μm pore size for 10 times and then through a polycarbonate membrane filter of 0.05 μm pore size for 24 times, whereby lead particles were prepared.

To 0.02 mL of the obtained suspension of lead particles, 0.01 mL of a 15 mg/mL aqueous solution of ODN was added, whereby complex particles were prepared.

Example 3

DOTAP, PEG-DSPE and distilled water were mixed such that the ratio of DOTAP/PEG-DSPE/distilled water was 30 mg/12 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.

To 0.02 mL of the obtained suspension of lead particles, 0.01 mL of a 15 mg/mL aqueous solution of ODN was added, whereby complex particles were prepared.

Example 4

DOTAP, PEG-DSPE and distilled water were mixed such that the ratio of DOTAP/PEG-DSPE/distilled water was 30 mg/18 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.

To 0.02 mL of the obtained suspension of lead particles, 0.01 mL of a 15 mg/mL aqueous solution of ODN was added, whereby complex particles were prepared.

Example 5

DOTAP, PEG-DSPE and distilled water were mixed such that the ratio of DOTAP/PEG-DSPE/distilled water was 30 mg/24 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.

To 0.02 mL of the obtained suspension of lead particles, 0.01 mL of a 15 mg/mL aqueous solution of ODN was added, whereby complex particles were prepared.

Example 6

DOTAP, polyoxyethylene hydrogenated castor oil (HCO-60, manufactured by NOF Corporation) and distilled water were mixed such that the ratio of DOTAP/polyoxyethylene hydrogenated castor oil/distilled water was 30 mg/24 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.

To 0.02 mL of the obtained suspension of lead particles, 0.01 mL of a 15 mg/mL aqueous solution of ODN was added, whereby complex particles were prepared.

Comparative Example 1

DOTAP and distilled water were mixed such that the ratio of DOTAP/distilled water was 30 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.

To 0.02 mL of the obtained suspension of lead particles, 0.01 mL of a 15 mg/mL aqueous solution of ODN was added, whereby complex particles were prepared.

Example 7

Lead particles were prepared in the same manner as in Example 1. To 0.02 mL of the obtained suspension of lead particles, 0.005 mL of a 8 mg/mL aqueous solution of pCAG-RLuc plasmid was added, whereby complex particles were prepared.

Example 8

Lead particles were prepared in the same manner as in Example 2. To 0.02 mL of the obtained suspension of lead particles, 0.005 mL of a 8 mg/mL aqueous solution of pCAG-RLuc plasmid was added, whereby complex particles were prepared.

Example 9

Lead particles were prepared in the same manner as in Example 3. To 0.02 mL of the obtained suspension of lead particles, 0.005 mL of a 8 mg/mL aqueous solution of pCAG-RLuc plasmid was added, whereby complex particles were prepared.

Example 10

Lead particles were prepared in the same manner as in Example 4. To 0.02 mL of the obtained suspension of lead particles, 0.005 mL of a 8 mg/mL aqueous solution of pCAG-RLuc plasmid was added, whereby complex particles were prepared.

Example 11

Lead particles were prepared in the same manner as in Example 5. To 0.02 mL of the obtained suspension of lead particles, 0.005 mL of a 8 mg/mL aqueous solution of pCAG-RLuc plasmid was added, whereby complex particles were prepared.

Comparative Example 2

Lead particles were prepared in the same manner as in Comparative Example 1. To 0.02 mL of the obtained suspension of lead particles, 0.005 mL of a 8 mg/mL aqueous solution of pCAG-RLuc plasmid was added, whereby complex particles were prepared.

Test Example 1

For the respective complex particles obtained in Examples 1 to 11 and Comparative Examples 1 to 2, the average particles diameter of each complex particles was measured with a dynamic light scattering (DLS) measurement device (NanoZS, manufactured by Malvern Instruments).
The results are shown in Table 1.
| [Table 1] | |
| --- | --- |
| Average particles diameter (nm) | |
| Example 1 | 157 |
| Example 2 | 111 |
| Example 3 | 91 |
| Example 4 | 68 |
| Example 5 | 72 |
| Example 6 | 152 |
| Comparative Example 1 | 399 |
| Example 7 | 230 |
| Example 8 | 158 |
| Example 9 | 129 |
| Example 10 | 95 |
| Example 11 | 101 |
| Comparative Example 2 | 301 |

Because the complex particles prepared in Examples 1 to 11 had an average particles diameter of 300 nm or less, it is considered that aggregation was inhibited, however, the complex particles prepared in Comparative Examples 1 and 2 had an average particles diameter of more than 300 nm.

Example 12

DOTAP, PEG-DSPE (manufactured by Avanti, the same applies hereinafter) and distilled water were mixed such that the ratio of DOTAP/PEG-DSPE/distilled water was 30 mg/12 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared. To 0.04 mL of the obtained suspension of lead particles, 0.01 mL of a 2 mg/mL aqueous solution of pCAG-LacZ plasmid was added, whereby complex particles were prepared.

Comparative Example 3

DOTAP and distilled water were mixed such that the ratio of DOTAP/distilled water was 30 mg/mL, and the mixture was stirred by shaking with a vortex mixer. The obtained suspension was passed, at room temperature, through a polycarbonate membrane filter of 0.4 $\mu$m pore size for 4 times and through a polycarbonate membrane filter of 0.1 $\mu$m pore size for 10 times and then through a polycarbonate membrane filter of 0.05 $\mu$m pore size for 24 times, whereby lead particles were prepared.
To 0.04 mL of the obtained suspension of lead particles, 0.01 mL of a 2 mg/mL aqueous solution of pCAG-LacZ plasmid was added, whereby complex particles were prepared.

Test Example 2

Visual observation of formation of an aggregate of the respective complex particles prepared in Example 12 and Comparative Example 3 was carried out. The results are shown in Table 2.
| [Table 2] | | |
|---|---|---|
| | Presence or absence of aggregate | |
| | Before addition of plasmid | After addition of plasmid |
| Comparative Example 3 | Absence | Presence |
| Example 12 | Absence | Absence |

As can be seen from Table 2, as for the complex particles prepared in Example 12, even when the plasmid was added, formation of aggregate was not observed, however, as for the complex particles prepared in Comparative Example 3, formation of aggregate was observed.

Example 13

Lead particles were prepared in the same manner as in Example 12. To 0.5 mL of the obtained suspension of lead particles, 0.25 mL of a 0.5 mg/mL aqueous solution of pCAG-Lacz plasmid was added and 1 ml of ethanol was added thereto, whereby complex particles were prepared.
To the obtained suspension of complex particles, 0.25 mL of a solution in which EPC (manufactured by NOF Corporation, the same applies hereinafter) and PEG-DSPE, both of which were the coating layer components, were dissolved in ethanol such that the ratio of EPC/PEG-DSPE/ethanol was 120 mg/25 mg/mL was added, and then, 23 mL of distilled water was gradually added thereto to adjust the concentration of ethanol to be 5 % by volume or less, whereby coated complex particles were prepared. The obtained suspension of coated complex particles was subjected to ultracentrifugation (110, 000 × g at 25°C for 1 hour) and the supernatant was removed. A physiological saline solution was added thereto to resuspend the residue, whereby a preparation was obtained.

Example 14

Lead particles were prepared in the same manner as in Example 12. To 0.5 mL of the obtained suspension of lead particles, 0.25 mL of a 3 mg/mL aqueous solution of pCAG-LacZ plasmid was added, and 1 mL of ethanol was added thereto, whereby complex particles were prepared.
By using the obtained suspension of complex particles, a preparation was obtained through the same preparation process of the coated complex particles as in Example 13.

Example 15

Lead particles were prepared in the same manner as in Example 12. To 0.5 mL of the obtained suspension of lead particles, 0.125 mL of a 2 mg/mL aqueous solution of pCAG-LacZ plasmid and 0.125 mL of a 6 mg/mL aqueous solution of dextran sulfate (manufactured by Merck, the same applies hereinafter) were added, and 1 mL of ethanol was added thereto, whereby complex particles were prepared.
By using the obtained suspension of complex particles, a preparation was obtained through the same preparation process of the coated complex particles as in Example 13.

Example 16

Lead particles were prepared in the same manner as in Example 12. To 0.5 mL of the obtained suspension of lead particles, 0.125 mL of a 1 mg/mL aqueous solution of pCAG-LacZ plasmid and 0.125 mL of a 12 mg/mL aqueous solution of dextran sulfate were added, and 1 ml of ethanol was added thereto, whereby complex particles were prepared.
By using the obtained suspension of complex particles, a preparation was obtained through the same preparation process of the coated complex particles as in Example 13.

Example 17

Lead particles were prepared in the same manner as in Example 12. To 0.5 mL of the obtained suspension of lead particles, 0.125 mL of a 1 mg/mL aqueous solution of pCAG-LacZ plasmid and 0.125 mL of a 3 mg/mL aqueous solution of dextran sulfate were added, and 1 ml of ethanol was added thereto, whereby complex particles were prepared.
By using the obtained suspension of complex particles, a preparation was obtained through the same preparation process of the coated complex particles as in Example 13.

Example 18

Lead particles were prepared in the same manner as in Example 12. To 0.5 mL of the obtained suspension of lead particles, 0.125 mL of a 1 mg/mL aqueous solution of pCAG-LacZ plasmid and 0.125 mL of a 3 mg/mL aqueous solution of dextran fluorescein anionic (manufactured by Molecular Probes) were added, and 1 mL of ethanol was added thereto, whereby complex particles were prepared.
By using the obtained suspension of complex particles, a preparation was obtained through the same preparation process of the coated complex particles as in Example 13.

Test Example 3

For the respective preparations obtained in Examples 13 to 18, the average particles diameter of each coated fine particles was measured with a dynamic light scattering (DLS) measurement device (A model ELS-800, manufactured by Otsuka Electronics). The results are shown in Table 3.
| [Table 3] | |
| --- | --- |
| Average particles diameter (nm) | |
| Example 13 | 96 |
| Example 14 | 320 |
| Example 15 | 117 |
| Example 16 | 122 |
| Example 17 | 102 |
| Example 18 | 97 |

Because in any of the preparations obtained in Examples 13 to 18, the average particles diameter was 350 nm or less, it is considered that aggregation of complex particles during the production process of the coated complex particles was inhibited.

Test Example 4

For the respective preparations obtained in Example 13 and Examples 15 to 17, the recovery rates of plasmid and EPC to the charged amounts were obtained as follows.
Each preparation was diluted to 10-fold with water, and to 200 μL of this diluted solution, 200 μL of a 10 w/v% aqueous solution of Triton X-100 (manufactured by Wako Pure Chemical Industries Ltd., the same applies hereinafter) was added, and then, 200 μL of a 2 μg/mL aqueous solution of ethidium bromide (manufactured by Wako Pure Chemical Industries Ltd.) and 1400 μL of a physiological saline solution were added thereto. By measuring the fluorescence at an excitation wavelength of 580 nm and a fluorescence wavelength of 615 nm using a spectrofluorometer (Hitachi, F-4500), plasmid in each preparation was determined. Further, EPC in the preparation was determined by an enzymatic method using Phospholipid C-test Wako (manufactured by Wako Pure Chemical Industries Ltd., the same applies hereinafter). The recovery rates of plasmid and EPC were calculated using the following equations (1) and (2), respectively. The results are shown in Table 4.
[Equation 1]

$$\text{Recovery rate of plasmid (\%)} = A/C \times 100 \qquad (1)$$

$$\text{Recovery rate of EPC (\%)} = B/D \times 100 \qquad (2)$$

A: amount of plasmid in preparation (mg)
B: amount of EPC in preparation (mg)
C: amount of charged plasmid in Example (mg)
D: amount of charged EPC in Example (mg)

[Table 4]

|  | Recovery rate (%) | |
| --- | --- | --- |
|  | Plasmid | EPC |
| Example 13 | 72.9 | 38.4 |
| Example 15 | 74.7 | 68.4 |
| Example 16 | 98.3 | 66.8 |
| Example 17 | 64.5 | 47.1 |

As seen from Table 4, as for any of the preparations obtained in Examples 13 and 15 to 17, the recovery rate of plasmid are not lower than 50%, which is high, and coating of the complex particles with the coating lipid is favorable. Further, as for the preparations containing an adhesion-competitive agent obtained in Examples 15 to 17, the recovery rate of EPC is roughly not lower than 50%, which is high, and coating of the complex particles with the coating lipid is efficient, therefore it is more preferred.

Example 19

Lead particles were prepared in the same manner as in Example 12. To 0.5 mL of the obtained suspension of lead particles, 0.125 mL of a 2 mg/mL aqueous solution of siRNA and 0.125 mL of a 6 mg/mL aqueous solution of dextran sulfate were added, and 1 ml of ethanol was added thereto, whereby complex particles were prepared.
To the obtained suspension of complex particles, 0.25 mL of a solution in which EPC and PEG-DSPE, both of which were the coating layer components, were dissolved in ethanol such that the ratio of EPC/PEG-DSPE/ethanol was 120 mg/25 mg/mL was added, and then, 23 mL of distilled water was gradually added thereto to adjust the concentration of ethanol to be 5 % by volume or less, whereby coated complex particles were prepared. The obtained suspension of coated complex particles was subjected to ultracentrifugation (110,000 × g at 25°C for 1 hour) and the supernatant was removed. A physiological saline solution was added thereto, and a solution obtained by dissolving 50 parts by weight of PEG-DSPE (4% by volume of the suspension of complex particles) relative to 120 parts by weight of EPC in a small amount of ethanol was mixed therewith. Then, the mixture was heated at 70°C for 2 minutes, whereby a preparation was obtained.

Test Example 5

The average particles diameter of the coated fine particles in the preparation obtained in Example 19 were measured with a DLS measurement device (A model ELS-800, manufactured by Otsuka Electronics). The results are shown in Table 5.
[Table 5]

| | Average particles diameter (nm) |
|---|---|
| Example 19 | 105 |

Because in the preparation obtained in Example 19, the average particles diameter was 350 nm or less, it is considered that aggregation of complex particles during the production process of the coated complex particles was inhibited.

Test Example 6

In the preparation obtained in Example 19, the recovery rates of siRNA and EPC to the charged amounts were obtained as follows.
The preparation was diluted to 20-fold with water, and to 50 µL of this diluted solution, 50 µL of a 10 w/v% aqueous solution of Triton X-100 was added; and then, 400 µL of a physiological saline solution was added thereto. By measuring the fluorescence at an excitation wavelength of 485 nm and a fluorescence wavelength of 530 nm using a fluorescence microplate reader (WALLAC, ARVO™ SX1420 Multilabel counter), siRNA in the preparation was determined. Further, EPC in the preparation was determined by an enzymatic method using Phospholipid C-test Wako (manufactured by Wako Pure Chemical Industries Ltd.). The recovery rates of siRNA and EPC were calculated using the following equations (3) and (4), respectively. The results are shown in Table 6.
[Equation 2]

$$\text{Recovery rate of siRNA (\%)} = A/C \times 100 \qquad (3)$$

$$\text{Recovery rate of EPC (\%)} = B/D \times 100 \qquad (4)$$

A: amount of siRNA in preparation (mg)
B: amount of EPC in preparation (mg)
C: amount of charged siRNA in Example 8 (mg)
D: amount of charged EPC in Example 8 (mg)

[Table 6]

| | Recovery rate (%) | |
|---|---|---|
| | siRNA | EPC |
| Example 19 | 61.7 | 55.8 |

As seen from Table 6, as for the preparation obtained in Example 19, the recovery rate of siRNA is not lower than 50%, which is high, and coating of the complex particles with the coating lipid was favorable. Also, the recovery rate of EPC is not lower than 50%, which is high, and coating of the complex particles with the coating lipid was efficient.

Example 20

Lead particles were prepared in the same manner as in Example 3.

To 0.25 mL of the obtained suspension of lead particles, 0.125 mL of a 15 mg/mL aqueous solution of ODN was added, whereby complex particles were prepared.

To the obtained suspension of complex particles, 0.5 mL of ethanol was added, and further, 0.125 mL of a solution obtained by dissolving EPC, which was the coating layer component, in an ethanol to give a final concentration of 120 mg/mL was added thereto, and then, 11.5 mL of distilled water was gradually added thereto to adjust the concentration of ethanol to be 5 % by volume or less, whereby coated complex particles were prepared. The obtained suspension of coated complex particles was subjected to ultracentrifugation (110,000 × g at 25°C for 1 hour) and the supernatant was removed. Then, a phosphate buffered saline solution (PBS) was added thereto to resuspend the residue, whereby a preparation was obtained

Example 21

Complex particles were prepared in the same manner as in Example 20.

To the obtained suspension of complex particles, 0.5 mL of ethanol was added, and further 0.125 mL of a solution in which EPC and PEG-DSPE (manufactured by NOF Corporation, the same applies hereinafter), both of which were the coating layer components, were dissolved in ethanol such that the ratio of EPC/PEG-DSPE/ethanol was 120 mg/10 mg/mL was added thereto. Then, 11.5 mL of distilled water was gradually added thereto to adjust the concentration of ethanol to be 5 % by volume or less, whereby coated complex particles were prepared.

The obtained suspension of coated complex particles was subjected to ultracentrifugation (110,000 × g at 25°C for 1 hour) and the supernatant was removed. Then, PBS was added thereto to resuspend the residue, whereby a preparation was obtained.

Example 22

Complex particles were prepared in the same manner as in Example 20.

To the obtained suspension of complex particles, 0.5 mL of ethanol was added, and further 0.125 mL of a solution in which EPC and PEG-DSPE, both of which were the coating layer components, were dissolved in ethanol such that the ratio of EPC/PEG-DSPE/ethanol was 120 mg/25 mg/mL was added thereto. Then, 11.5 mL of distilled water was gradually added thereto to adjust the concentration of ethanol to be 5 % by volume or less, whereby coated complex particles were prepared.

The obtained suspension of coated complex particles was subjected to ultracentrifugation (110,000 × g at 25°C for 1 hour) and the supernatant was removed. Then, PBS was added thereto to resuspend the residue, whereby a preparation was obtained.

Example 23

Complex particles were prepared in the same manner as in Example 20.

To the obtained suspension of complex particles, 0.5 mL of ethanol was added, and further 0.125 mL of a solution in which EPC and PEG-DSPE, both of which were the coating layer components, were dissolved in ethanol such that the ratio of EPC/PEG-DSPE/ethanol was 120 mg/50 mg/mL was added thereto. Then, 11.5 mL of distilled water was gradually added thereto to adjust the concentration of ethanol to be 5 % by volume or less, whereby coated complex particles were prepared.

The obtained suspension of coated complex particles was subjected to ultracentrifugation (110,000 × g at 25°C for 1 hour) and the supernatant was removed. Then, PBS was added thereto to resuspend the residue, whereby a preparation was obtained.

Example 24

Lead particles were prepared in the same manner as in Example 3.

To 0.25 mL of the obtained suspension of lead particles, 0.0625 mL of a 2 mg/mL aqueous solution of pCAG-RLuc plasmid and 0.0625 mL of a 20 mg/mL aqueous solution of dextran sulfate were added, whereby complex particles were prepared.

To the obtained suspension of complex particles, 0.5 mL of ethanol was added, and further 0.125 mL of a solution in which EPC as the coating layer component, was dissolved in ethanol to give a final concentration of 120 mg/mL was added thereto. Then, 11.5 mL of distilled water was gradually added thereto to adjust the concentration of ethanol to be 5 % by volume or less, whereby coated complex particles were prepared. The obtained suspension of coated complex particles was subjected to ultracentrifugation (110,000 × g at 25°C for 1 hour) and the supernatant was removed. Then, a physiological saline solution was added thereto to resuspend the residue, whereby a preparation was obtained.

Example 25.

Complex particles were prepared in the same manner as in Example 24.
To the obtained suspension of complex particles, 0.5 mL of ethanol was added, and further 0.125 mL of a solution in which EPC and PEG-DSPE, both of which were the coating layer components, were dissolved in ethanol such that the ratio of EPC/PEG-DSPE/ethanol was 120 mg/10 mg/mL was added thereto. Then, 11.5 mL of distilled water was gradually added thereto to adjust the concentration of ethanol to be 5 % by volume or less, whereby coated complex particles were prepared.
The obtained suspension of coated complex particles was subjected to ultracentrifugation (110,000 × g at 25°C for 1 hour) and the supernatant was removed. Then, a physiological saline solution was added thereto to resuspend the residue, whereby a preparation was obtained.

Example 26

Complex particles were prepared in the same manner as in Example 24.
To the obtained suspension of complex particles, 0.5 mL of ethanol was added, and further 0.125 mL of a solution in which EPC and PEG-DSPE, both of which were the coating layer components, were dissolved in ethanol such that the ratio of EPC/PEG-DSPE/ethanol was 120 mg/25 mg/mL was added thereto. Then, 11.5 mL of distilled water was gradually added thereto to adjust the concentration of ethanol to be 5 % by volume or less, whereby coated complex particles were prepared.
The obtained suspension of coated complex particles was subjected to ultracentrifugation (110,000 × g at 25°C for 1 hour) and the supernatant was removed. Then, a physiological saline solution was added thereto to resuspend the residue, whereby a preparation was obtained.

Example 27

Complex particles were prepared in the same manner as in Example 24.
To the obtained suspension of complex particles, 0.5 mL of ethanol was added, and further 0.125 mL of a solution in which EPC and PEG-DSPE, both of which were the coating layer components, were dissolved in ethanol such that the ratio of EPC/PEG-DSPE/ethanol was 120 mg/50 mg/mL was added thereto. Then, 11.5 mL of distilled water was gradually added thereto to adjust the concentration of ethanol to be 5 % by volume or less, whereby coated complex particles were prepared.
The obtained suspension of coated complex particles was subjected to ultracentrifugation (110,000 × g at 25°C for 1 hour) and the supernatant was removed. Then, a physiological saline solution was added thereto to resuspend the residue, whereby a preparation was obtained.

Test Example 7

For the respective preparations obtained in Examples 20 to 27, the average particles diameter of each coated fine particles was measured with a DLS measurement device (NanoZS, manufactured by Malvern Instruments). The results are shown in Table 7.
[Table 7]

| | Average particles diameter (nm) |
|---|---|
| Example 20 | 143 |
| Example 21 | 120 |
| Example 22 | 113 |
| Example 23 | 116 |

| (continued) | |
| --- | --- |
| | Average particles diameter (nm) |
| Example 24 | 138 |
| Example 25 | 131 |
| Example 26 | 131 |
| Example 27 | 138 |

Because in the preparations obtained in Examples 20 to 27, the average particles diameter was 350 nm or less, it is considered that aggregation of complex particles during the production process of the coated complex particles was inhibited.

Test Example 8

In the respective preparations obtained in Examples 20 to 27, the recovery rates of EPC to the charged amounts were obtained in the same manner as in Test Example 4. The results are shown in Table 8
| [Table 8] | |
| --- | --- |
| | Recovery rate of EPC (%) |
| Example 20 | 57.8 |
| Example 21 | 63.5 |
| Example 22 | 54.8 |
| Example 23 | 29.7 |
| Example 24 | 47.0 |
| Example 25 | 62.2 |
| Example 26 | 65.0 |
| Example 27 | 39.3 |

As can be seen from Table 8, in any of the preparations obtained in Examples 20 to 27, the recovery rate of EPC is high, and coating of the complex particles with the coating lipid was efficiently carried out. Further, the preparations obtained in Examples 21, 22, 25 and 26, in which the ratio of the water-soluble polymer derivative to the total coating layer components is 1:0.25 to 1:0.01 in ratio by weight, were more preferred because the average particles diameters of the coated fine particles were smaller, and the recovery rates of EPC were higher.

Industrial Applicability

According to the present invention, a method of inhibiting aggregation of complex particles in which a drug is adhered to lead particles and a method of producing the complex particles and the like are provided, and further, a method of producing coated complex particles in which aggregation-inhibited complex particles are coated with a coating layer, coated complex particles that can be produced by the production method and the like are provided.

**Claims**

1. A method of inhibiting aggregation of complex particles in which a drug is adhered to lead particles, **characterized by** containing a lipid derivative or a fatty acid derivative of one or more substance(s) selected from sugars, peptides, nucleic acids and water-soluble polymers or a surfactant in the lead particles.

2. The method of inhibiting aggregation of complex particles according to claim 1, wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

3. The method of inhibiting aggregation of complex particles according to claim 1, wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

4. The method of inhibiting aggregation of complex particles according to any one of claims 1 to 3, wherein the complex particles in which a drug is adhered to lead particles are complex particles obtained by dispersing or dissolving the drug so as to be contained in a liquid in which the lead particles are dispersed and allowing the drug adhered to the lead particles.

5. The method of inhibiting aggregation of complex particles according to any one of claims 1 to 4, wherein the complex particles in which a drug is adhered to lead particles are complex particles in which a drug is electrostatically adhered to lead particles.

6. The method of inhibiting aggregation of complex particles according to any one of claims 1 to 5, wherein the lead particles are lead particles having electrostatic charge opposite to that of the drug.

7. The method of inhibiting aggregation of complex particles according to any one of claims 1 to 6, wherein the lead particles are fine particles containing as a constituent component liposome containing a lipid with electrostatic charge opposite to that of the drug.

8. The method of inhibiting aggregation of complex particles according to any one of claims 1 to 7, wherein the drug is a nucleic acid.

9. The method of inhibiting aggregation of complex particles according to claim 8, wherein the nucleic acid as the drug is one or more substance(s) selected from genes, DNA, RNA, oligonucleotides, plasmids and siRNA.

10. The method of inhibiting aggregation of complex particles according to any one of claims 1 to 9, wherein the complex particles in which a drug is adhered to lead particles are complex particles in which a drug and an adhesion-competitive agent are adhered to lead particles.

11. The method of inhibiting aggregation of complex particles according to claim 10, wherein the complex particles in which a drug and an adhesion-competitive agent are adhered to lead particles are complex particles obtained by dispersing or dissolving the drug and the adhesion-competitive agent so as to be contained in a liquid in which the lead particles are dispersed and allowing the drug and the adhesion-competitive agent adhered to the lead particles.

12. The method of inhibiting aggregation of complex particles according to claim 10 or 11, wherein the complex particles in which a drug and an adhesion- competitive agent are adhered to lead particles are complex particles in which a drug and an adhesion-competitive agent are electrostatically adhered to lead particles.

13. The method of inhibiting aggregation of complex particles according to any one of claims 10 to 12, wherein the adhesion-competitive agent is one or more substance(s) selected from lipids, surfactants, nucleic acids, proteins, peptides and polymers.

14. The method of inhibiting aggregation of complex particles according to any one of claims 10 to 12, wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.

15. An inhibitor for aggregation of complex particles in which a drug is adhered to lead particles, containing a lipid derivative or a fatty acid derivative of one or more substance(s) selected from sugars, peptides, nucleic acids and water-soluble polymers or a surfactant.

16. The inhibitor for aggregation of complex particles according to claim 15, wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

17. The inhibitor for aggregation of complex particles according to claim 15, wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

18. A method of producing complex particles in which a nucleic acid as a drug adhered to lead particles, comprising the step of dispersing or dissolving the nucleic acid as a drug so as to be contained in a liquid in which the lead particles containing a lipid derivative or a fatty acid derivative of one or more substance(s) selected from sugars, peptides, nucleic acids and water-soluble polymers or a surfactant are dispersed, thereby allowing the nucleic acid as a drug adhered to the lead particles.

19. A method of producing complex particles in which a drug is adhered to lead particles, comprising the step of dispersing or dissolving the drug and an adhesion-competitive agent so as to be contained in a liquid in which the lead particles containing a lipid derivative or a fatty acid derivative of one or more substance(s) selected from sugars, peptides, nucleic acids and water-soluble polymers or a surfactant are dispersed, thereby allowing the drug and the adhesion-competitive agent adhered to the lead particles.

20. The method of producing complex particles according to claim 19, wherein the adhesion-competitive agent is one or more substance(s) selected from lipids, surfactants, nucleic acids, proteins, peptides and polymers.

21. The method of producing complex particles according to claim 19, wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.

22. The method of producing complex particles according to any one of claims 19 to 21, wherein the drug is a nucleic acid.

23. The method of producing complex particles according to claim 18 or 22, wherein the nucleic acid as the drug is one or more substance(s) selected from genes, DNA, RNA, oligonucleotides, plasmids and siRNA.

24. The method of producing complex particles according to any one of claims 18 to 23, wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

25. The method of producing complex particles according to any one of claims 18 to 23, wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

26. The method of producing complex particles according to any one of claims 18 to 25, wherein the lead particles are lead particles having electrostatic charge opposite to that of the drug.

27. The method of producing complex particles according to any one of claims 18 to 25, wherein the lead particles are fine particles containing as a constituent component liposome containing a lipid with electrostatic charge opposite to that of the drug.

28. Complex particles which can be produced by the method of producing complex particles according to any one of claims 18 to 27.

29. Complex particles comprising:

lead particles containing a lipid derivative or a fatty acid derivative of one or more substance(s) selected from sugars, peptides, nucleic acids and water-soluble polymers or a surfactant; and
a nucleic acid as a drug adhered to the lead particles.

30. Complex particles comprising:

lead particles containing a lipid derivative or a fatty acid derivative of one or more substance(s) selected from sugars, peptides, nucleic acids and water-soluble polymers or a surfactant;
a drug adhered to the lead particles; and
an adhesion-competitive agent adhered to the lead particles.

31. The complex particles according to claim 30, wherein the adhesion-competitive agent is one or more substance(s) selected from lipids, surfactants, nucleic acids, proteins, peptides and polymers.

32. The complex particles according to claim 30, wherein the adhesion-competitive agent is one or more substance(s) selected from dextran sulfate, sodium dextran sulfate, chondroitin sulfate, sodium chondroitin sulfate, hyaluronic acid, chondroitin, dermatan sulfate, heparan sulfate, heparin, ketaran sulfate and dextran fluorescein anionic.

33. The complex particles according to any one of claims 30 to 32, wherein the drug is a nucleic acid.

34. The complex particles according to claim 29 or 33, wherein the nucleic acid as the drug is one or more substance(s) selected from genes, DNA, RNA, plasmids and siRNA.

35. The complex particles according to any one of claims 29 to 34, wherein the lipid derivative or the fatty acid derivative of one or more substance(s) selected from sugars, peptides, nucleic acids and water-soluble polymers or the surfactant is a lipid derivative or a fatty acid derivative of a water-soluble polymer.

36. The complex particles according to any one of claims 29 to 34, wherein the lipid derivative or the fatty acid derivative of one or more substance(s) selected from sugars, peptides, nucleic acids and water-soluble polymers or the surfactant is one or more substance(s) selected from polyethylene glycolated lipids, polyethylene glycol sorbitan fatty acid esters, polyethylene glycol fatty acid esters, polyglycerolated lipids, polyglycerol fatty acid esters, polyoxyethylene polypropylene glycol, glycerol fatty acid esters and polyethylene glycol alkyl ethers.

37. The complex particles according to any one of claims 29 to 36, wherein the lead particles are lead particles having electrostatic charge opposite to that of the drug.

38. The complex particles according to any one of claims 29 to 36, wherein the lead particles are fine particles containing as a constituent component liposome containing a lipid with electrostatic charge opposite to that of the drug.

39. A method of producing coated complex particles comprising the steps of:

preparing a liquid (liquid A) containing a polar organic solvent in which the complex particles according to any one of claims 28 to 38 are dispersed and a coating layer component is dissolved; and
coating the complex particles with a coating layer composed of the coating layer component by reducing the ratio of the polar organic solvent in the liquid A.

40. The method of producing coated complex particles according to claim 39, wherein the step of preparing the liquid A comprises the steps of:

preparing a liquid (liquid B) containing a polar organic solvent in which the complex particles according to any one of claims 28 to 38 are dispersed;
preparing a liquid (liquid C) obtained by dissolving the coating layer component in a solvent containing a polar organic solvent which is the same as or different from that in the liquid B; and
mixing the liquid B and the liquid C.

41. The method of producing coated complex particles according to claim 39 or 40, wherein the coating layer is a lipid membrane.

42. The method of producing coated complex particles according to claim 41, wherein the coating layer is a coating layer containing a water-soluble polymer derivative.

43. Coated complex particles which can be produced by the method of producing coated complex particles according to any one of claims 39 to 42.

44. Coated complex particles comprising the complex particles according to any one of claims 28 to 38 and a coating layer for coating the complex particles, wherein in a solvent containing a polar solvent at a concentration within a range where the complex particles are not dissolved and can be dispersed therein, a coating layer component constituting the coating layer is dissolved when the concentration of the polar solvent is relatively high, and is deposited or assembled when the concentration of the polar solvent is relatively low.

45. The coated complex particles according to claim 44, wherein the coating layer is a lipid membrane.

46. The coated complex particles according to claim 45, wherein the coating layer is a coating layer containing a water-soluble polymer derivative.

47. The coated complex particles according to any one of claims 44 to 46, wherein the average particles diameter of the coated complex particles are 300 nm or less.

## INTERNATIONAL SEARCH REPORT

| International application No. |
| --- |
| PCT/JP2005/004241 |

A. CLASSIFICATION OF SUBJECT MATTER
   Int.Cl⁷ A61K47/26, 9/10, 9/127, 31/7088, 31/713, 47/30, 47/36,
             47/42, 47/44, A61P35/00

According to International Patent Classification (IPC) or to both national classification and IPC

B. FIELDS SEARCHED

Minimum documentation searched (classification system followed by classification symbols)
   Int.Cl⁷ A61K47/26, 9/10, 9/127, 31/7088, 31/713, 47/30, 47/36,
             47/42, 47/44, A61P35/00

Documentation searched other than minimum documentation to the extent that such documents are included in the fields searched

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| X | WO 2002/028367 A1 (Kyowa Hakko Kogyo Co., Ltd.), 11 April, 2002 (11.04.02), Claims 1 to 3, 8 to 14, 19; page 6, line 8 to page 9, line 19; page 13, line 3 to page 14, line 12; page 15, lines 5 to 11 & EP 1323415 A1          & US 2004/0022938 A1 | 1-47 |
| A | JP 2002-501511 A (Inex Pharmaceuticals Corp.), 15 January, 2002 (15.01.02), Claims; page 26, line 25 to page 27 & WO 98/51278 A2          & EP 1027033 A2 | 1-47 |

☐ Further documents are listed in the continuation of Box C.          ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 22 June, 2005 (22.06.05) | 09 August, 2005 (09.08.05) |

| Name and mailing address of the ISA/ | Authorized officer |
| --- | --- |
| Japanese Patent Office | |
| Facsimile No. | Telephone No. |

Form PCT/ISA/210 (second sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2002508765 T
- JP 2002501511 T
- WO 0228367 A
- JP 2572554 B
- JP 2659136 B
- WO 0133957 A

### Non-patent literature cited in the description

- *Biochimica et Biophysica Acta,* 2001, vol. 1510, 152-166
- *Biochemical and Biophysical Research Communication,* 2002, vol. 296, 1000-1004
- *Biochimica et Biophysica Acta,* 1996, vol. 1281, 139-149
- *Journal of Controlled Release,* 1996, vol. 41, 121-130
- *Journal of Molecular Biology,* 1965, vol. 13, 238-252
- *Journal of Cell Biology,* 1975, vol. 66, 621-634
- *FEBS Letters,* 1979, vol. 99, 210-214
- *Archives of Biochemistry and Biophysics,* 1981, vol. 212, 186-194
- *Proceedings of the National Academy of Science United States of America,* 1978, vol. 75, 4194-4198
- Stealth Liposome. CRC Press Inc, 1995, 93-102
- *Emulsion and Nanosuspensions for the Formulation of Poorly Soluble Drugs,* 1998, 267-294
- *Proceedings of the National Academy of Science United States of America,* 1972, vol. 69, 2110-2114